(19) Europäisches Patentamt / European Patent Office / Office européen des brevets

(11) **EP 3 765 848 B1**

(12) **EUROPEAN PATENT SPECIFICATION**

(45) Date of publication and mention of the grant of the patent:
**12.02.2025 Bulletin 2025/07**

(21) Application number: **19717017.8**

(22) Date of filing: **12.03.2019**

(51) International Patent Classification (IPC):
*G01N 33/50* *(2006.01)*

(52) Cooperative Patent Classification (CPC):
**G01N 33/5067**

(86) International application number:
**PCT/US2019/021771**

(87) International publication number:
**WO 2019/178039 (19.09.2019 Gazette 2019/38)**

(54) **HIGH DENSITY 3D HEPATOCYTE SPHEROID PLATFORM FOR DRUG ADME STUDIES**

3D-HEPATOCYTE-SPHEROID-PLATTFORM MIT HOHER DICHTE FÜR DROGEN-ADME-STUDIEN

PLATE-FORME DE SPHÉROÏDES D'HÉPATOCYTES 3D HAUTE DENSITÉ POUR ÉTUDES ADME DE MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.03.2018 US 201862642447 P**

(43) Date of publication of application:
**20.01.2021 Bulletin 2021/03**

(73) Proprietor: **Discovery Life Sciences, LLC**
**Huntsville, AL 35806 (US)**

(72) Inventors:
• **GORAL, Vasiliy Nikolaevich**
**New York 14870 (US)**
• **LI, Feng**
**Shrewsbury, Massachusetts 01545 (US)**
• **MARTIN, Gregory Roger**
**Acton, Maine 04001 (US)**
• **TANNER, Allison Jean**
**Portsmouth, New Hampshire 03801 (US)**
• **ZUO, Rongjun**
**Roxbury, Massachusetts 02132 (US)**

(74) Representative: **Elkington and Fife LLP**
**Prospect House**
**8 Pembroke Road**
**Sevenoaks, Kent TN13 1XR (GB)**

(56) References cited:
WO-A1-2014/179196      WO-A1-2016/069895
WO-A1-2017/083727      WO-A2-2006/127768

• LI FENG ET AL: "Corning  HepatoCells Spheroids: A New High Throughput 3D Model for Hepatotoxicity Studies Application Note", 1 January 2015 (2015-01-01), pages 1 - 6, XP055947812, Retrieved from the Internet <URL:https://www.corning. com/catalog/cls/documents/application-note s/Application_Note_CLS-DL-AN-340_Cornin g_HepatoCells_Spheroids.pdf> [retrieved on 20220801]
• GUIDE USER: "Corning  Spheroid Microplates", 1 January 2014 (2014-01-01), pages 1 - 6, XP055947856, Retrieved from the Internet <URL:https://www.corning. com/catalog/cls/documents/protocols/CL S-AN-235.pdf> [retrieved on 20220801]
• MATTHEW G. BARON ET AL: "Pharmaceutical Metabolism in Fish: Using a 3-D Hepatic In Vitro Model to Assess Clearance", PLOS ONE, vol. 12, no. 1, 3 January 2017 (2017-01-03), pages e0168837, XP055587665, DOI: 10.1371/ journal.pone.0168837

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- TAKAKO OHKURA ET AL: "Evaluation of Human Hepatocytes Cultured by Three-dimensional Spheroid Systems for Drug Metabolism", DRUG METABOLISM AND PHARMACOKINETICS, vol. 29, no. 5, 1 January 2014 (2014-01-01), JP, pages 373 - 378, XP055587666, ISSN: 1347-4367, DOI: 10.2133/dmpk.DMPK-13-RG-105

- KE HU ET AL: "Primary Hepatocytes Cultured on a Fiber-Embedded PDMS Chip to Study Drug Metabolism", POLYMERS, vol. 9, no. 12, 10 June 2017 (2017-06-10), pages 215, XP055587650, DOI: 10.3390/polym9060215

**Description**

**CROSS-REFERENCE TO RELATED APPLICATIONS**

This application claims the benefit of priority under 35 U.S.C. §120 of U.S. Provisional Application Serial No. 62/642447 filed on March 13, 2018.

**FIELD**

The present disclosure generally relates to methods for evaluating the interaction of a candidate compound with 3D hepatocyte spheroids in an *in vitro* culture, including evaluating the metabolism of a candidate compound, for use in various biochemical and molecular biology studies. The methods are performed in labware that combine 3D spheroid culture with micro-patterned design that allows for prolonged maintenance of liver cells (e.g. hepatocytes) viability and functionality, but also allows for multiple to several hundreds of spheroids to be treated under the same conditions and for the production of sufficient materials (e.g., parent drug, drug metabolites, and DNA, RNA, and proteins from cells) and higher detection signal intensity for ADME/Tox (absorption, distribution, metabolism, excretion and toxicity) and other related studies for more accurate detection. The methods allow for, among other uses, the investigation and generation of accurate *in vitro* intrinsic clearance data for compounds, and thus more accurate prediction of *in vivo* clearance, particularly with low clearance compounds.

**TECHNICAL BACKGROUND**

PLOS ONE, (20170103), vol. 12, no. 1, MATTHEW G. BARON ET AL discloses pharmaceutical Metabolism in Fish: Using a 3-D Hepatic In Vitro Model to Assess Clearance. ADME/Tox (absorption, distribution, metabolism, excretion and toxicity) properties of a compound are critical elements for predicting the compound's clinical success. Early ADME/Tox screenings are used during the drug discovery process to select against drugs with problematic profiles, reduce metabolic liability, and to ultimately enable desired dosing regimen. An increase in low clearance compounds in pharmaceutical drug discovery programs presents challenges to conventional *in vitro* systems for clearance studies. The ability to use *in vitro* data to predict *in vivo* hepatic clearance of a compound is essential in drug discovery and development, as understanding a compound's intrinsic clearance parameter is the most important parameter for determining drug-half-life, oral bioavailability, dose, and dosing regimens. Further, an unexpected *in vivo* clearance of a drug candidate can lead to exposure issues and safety concerns. However, current *in vitro* liver models cannot reliably predict the *in vivo* clearance or half-life of low clearance drug candidates due to the detection limits of these models.

Accordingly, on-going need exists for alternative *in vitro* models and methods to enable the investigation the ADME/Tox properties for compounds, and more particularly for *in vitro* models and methods that can reliably and accurately predict the *in vivo* clearance or half-life of low clearance compounds.

**SUMMARY**

The invention is defined in the appended claims.

In accordance with various embodiments of the present disclosure, methods and labware for evaluating the interaction of a candidate compound with 3D hepatocyte spheroids in an *in vitro* culture, for use in various biochemical and molecular biology studies, particularly ADME/Tox (absorption, distribution, metabolism, excretion and toxicity) studies. In aspects, the methods and labware can be used to evaluate the metabolism of a candidate compound in an *in vitro* 3D hepatocyte spheroid culture. The methods allow for, among other uses, the investigation and generation of accurate *in vitro* intrinsic clearance data of compounds, and thus more accurate prediction *of in vivo* clearance, particularly with low clearance candidate compounds.

In various embodiments, an assay method for evaluating the interaction of one or more low clearance candidate compounds with hepatocytes is disclosed. The assay method includes culturing hepatocytes in a cell culture article to form a spheroid, wherein the cell culture article comprises a chamber. The chamber is a well of a multi-well plate. In embodiments, at the bottom of each well is an array of microcavities. Each microcavity is structured to constrain the hepatocytes to grow in a 3D spheroid conformation. The assay method further includes contacting the 3D spheroid hepatocytes with one or more low clearance candidate compounds. The assay method also includes measuring the *in vitro* intrinsic clearance of the one or more low clearance candidate compounds. In embodiments, the culture is a long-term culture. In some embodiments, the long-term culture is at least about 12 hours, at least about 24 hours, at least about 48 hours, at least about 72 hours, at least about 96 hours, at least about 7 days, at least about 14 days, at least about 21 days, or at least about 28 days.

In some embodiments, each microcavity of the chamber of the cell culture article includes a top aperture and a liquid impermeable bottom comprising a bottom surface. In embodiments, at least a portion of the bottom surface includes a low-adhesion or no-adhesion material in or on the bottom surface. In some embodiments, the liquid impermeable bottom including the bottom surface is gas-permeable. In some embodiments, at least a portion of the bottom is transparent.

In some embodiments, the bottom surface comprises a concave bottom surface. In some embodiments, the at least one concave surface of each microcavity of the chamber includes a hemi-spherical surface, a conical surface having a taper of 30 to about 60 degrees from the side walls to the bottom surface, or a combination thereof.

In some embodiments, the chamber further comprises a side wall. In some embodiments, the side wall of the chamber includes a vertical cylinder, a portion of a vertical conic of decreasing diameter from the chamber's top to bottom surface, a vertical square shaft having a conical transition to the at least one concave bottom surface, or a combination thereof.

In some embodiments, each microcavity comprises a side wall. In some embodiments, the side wall of each microcavity includes a vertical cylinder, a portion of a vertical conic of decreasing diameter from the chamber's top to bottom surface, a vertical square shaft having a conical transition to the at least one concave bottom surface, or a combination thereof.

In some embodiments, the cell culture article includes from 1 to about 2,000 of said chambers, wherein each chamber is physically separated from any other chamber. In embodiments the chambers are wells of a multi-well plate. For example, a cell culture article may have 6, 12, 24, 96, 384 or 1536 chambers. In some embodiments, each chamber includes from about 25 to about 1,000 of said microcavities.

In some embodiments, the *in vitro* intrinsic clearance of the one or more low clearance candidate compounds is measured by disappearance of the one or more candidate compounds. In some embodiments, the *in vitro* intrinsic clearance of the one or more candidate compounds is measured by the formation of metabolites from the one or more candidate compounds.

In some embodiments, the measured *in vitro* intrinsic clearance of the one or more candidate compounds is utilized to predict *in vivo* half-life of the one or more candidate compounds. In some embodiments, the measured *in vitro* intrinsic clearance of the one or more candidate compounds is utilized to predict *in vivo* clearance of the one or more candidate compounds.

In some embodiments, the assay method further comprises the step of analyzing metabolites of the one or more low clearance candidate compounds, wherein the metabolites are generated during the incubation of the 3D spheroid hepatocytes with the one or more low clearance candidate compounds. In some embodiments, analyzing metabolites of the one or more candidate compounds comprises identification of metabolites of the one or more candidate compounds generated during the incubation of the 3D spheroid hepatocytes with the one or more low clearance candidate compounds. In some embodiments, analyzing metabolites of the one or more candidate compounds comprises quantification of metabolites of the one or more candidate compounds generated during the incubation of the 3D spheroid hepatocytes with the one or more low clearance candidate compounds.

In some embodiments, the assay further method comprises the step of analyzing the molecular, biochemical, or genetic effects of the one or more low clearance candidate compounds on the 3D spheroid hepatocytes. In some embodiments, analyzing the molecular, biochemical, or genetic effects of the one or more low clearance candidate compounds on the 3D spheroid hepatocytes comprises measuring gene and/or protein expression change during the incubation of the 3D spheroid hepatocytes with the one or more low clearance candidate compounds. In some embodiments, analyzing the molecular, biochemical, or genetic effects of the one or low clearance candidate compounds on the 3D spheroid hepatocytes comprises measuring DNA, RNA, and/or proteins produced by the 3D spheroid hepatocytes (e.g., isolated from the cells, cell extracts, and/or or media) during the incubation of the 3D spheroid hepatocytes with the one or more low clearance candidate compounds.

In some embodiments, the hepatocytes include primary human hepatocytes. In some embodiments, the hepatocytes include a hepatic cell line.

In some embodiments, the assay method further includes evaluating a plurality of candidate compounds simultaneously.

In some embodiments, the 3D hepatocyte spheroids are functional stable for at least three weeks. In some embodiments, the functional stability of the 3D hepatocyte spheroids is determined by measuring metabolic activity, cell function, gene expression, or a combination thereof. In some embodiments, the functional stability of the 3D hepatocyte spheroids is measured by CYP3A4 activity.

Additional features and advantages of the subject matter of the present disclosure will be set forth in the detailed description which follows, and in part will be readily apparent to those skilled in the art from that description or recognized by practicing the subject matter of the present disclosure as described herein, including the detailed description which follows, the claims, as well as the appended drawings.

It is to be understood that both the foregoing general description and the following detailed description present embodiments of the subject matter of the present disclosure, and are intended to provide an overview or framework for understanding the nature and character of the subject matter of the present disclosure as it is claimed. The accompanying drawings are included to provide a further understanding of the subject matter of the present disclosure, and are incorporated into and constitute a part of this specification. The drawings illustrate various embodiments of the subject matter of the present disclosure and together with the description serve to explain the principles and operations of the subject matter of the present disclosure. Additionally, the drawings and descriptions are meant to be merely illustrative, and are not intended to limit the scope of the claims in any manner.

## DESCRIPTION OF THE FIGURES

The following detailed description of specific embodiments of the present disclosure can be best understood when read in conjunction with the following drawings, where like structure is indicated with like reference numerals and in which:

**FIG. 1**A**, FIG. 1B** and **FIG. 1C** show an embodiment of a multi-well microplate, in this case a 96-well spheroid microplate, having an array of microcavities on the bottom surface of each well to provide multiple spheroids in each of the 96 wells. **FIG. 1**A shows a multi-well microplate. **FIG. 1B** shows illustrates a single well of the multi-well plate. **FIG. 1C** is an exploded view of the area of the bottom surface of the single well shown in the box C in **FIG. 1**B**.**

**FIG. 2A** is an illustration of an exemplary array of microcavities. **FIG. 2B** is an illustration of an additional exemplary array of microcavities.

**FIG. 3A** is a photograph of Corning® HepatoCells spheroids when the spheroids were first manually pooled together at 0 hours. **FIG. 3B** is a photograph showing fusion of Corning HepatoCells® spheroid when the spheroids were manually pooled together and incubated for 22 hours.

**FIG. 4** is an embodiment of the method disclosed herein.

**FIG. 5** is a graph showing CYP3A4 activity of two lots of 3D hepatocyte spheroids cultured in a 96-well spheroid plate as compared to the CYP3A4 activity of hepatocytes cultured in 2D.

**FIG. 6**A-I are graphs showing *in vitro* measurement of classic low clearance compounds using an embodiment of the methods disclosed herein.

**FIG. 7**A is an illustration showing an microcavity insert. **FIG. 7**B is a photograph of primary human hepatocytes cultured in a 24 well microcavity insert at day 1. **FIG. 7**C is a photograph of primary human hepatocytes cultured in a 24 well microcavity insert at day 3, demonstrating 3D spheroid formation.

**FIG. 8**A-I are graphs showing *in vitro* measurement of classic low clearance compounds using suspension cells.

## DETAILED DESCRIPTION

Reference will now be made in greater detail to various embodiments of the subject matter of the present disclosure, some embodiments of which are illustrated in the accompanying drawings. Like numbers used in the figures refer to like components, steps and the like. However, it will be understood that the use of a number to refer to a component in a given figure is not intended to limit the component in another figure labeled with the same number. In addition, the use of different numbers to refer to components is not intended to indicate that the different numbered components cannot be the same or similar to other numbered components.

The following description of particular embodiment(s) is merely exemplary in nature and is in no way intended to limit the scope of the invention, its application, or uses, which may, of course, vary. The invention is described with relation to the non-limiting definitions and terminology included herein. These definitions and terminology are not designed to function as a limitation on the scope or practice of the invention but are presented for illustrative and descriptive purposes only. Unless otherwise defined, all terms (including technical and scientific terms) used herein have the same meaning as commonly understood by one of ordinary skill in the art to which this disclosure belongs. It will be further understood that terms such as those defined in commonly used dictionaries, should be interpreted as having a meaning that is consistent with their meaning in the context of the relevant art and the present disclosure, and will not be interpreted in an idealized or overly formal sense unless expressly so defined herein.

## DEFINITIONS

As used herein, singular forms "a," "an" and "the" include plural referents unless the context clearly dictates otherwise. Thus, for example, reference to a "structured bottom surface" includes examples having two or more such "structured bottom surfaces" unless the context clearly indicates otherwise.

As used in this specification and the appended claims, the term "or" is generally employed in its sense including "and/or" unless the content clearly dictates otherwise. The term "and/or" means one or all of the listed elements or a combination of any two or more of the listed elements.

As used herein, "have", "has", "having", "include", "includes", "including", "comprise", "comprises", "comprising" or the like are used in their open ended inclusive sense, and generally mean "include, but not limited to", "includes, but not limited to", or "including, but not limited to."

"Optional" or "optionally" means that the subsequently described event, circumstance, or component, can or cannot occur, and that the description includes instances where the event, circumstance, or component, occurs and instances where it does not.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the inventive technology.

Ranges can be expressed herein as from "about" one particular value, and/or to "about" another particular value. When such a range is expressed, examples include from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

Also herein, the recitations of numerical ranges by endpoints include all numbers subsumed within that range (e.g., 1 to 5 includes 1, 1.5, 2, 2.75, 3, 3.80, 4, 5, etc.). It should be further understood that every numerical range given throughout this specification will include every narrower numerical range that falls within such broader numerical range, as if such narrower numerical ranges were all expressly written herein. Where a range of values is "greater than", "less than", etc. a particular value, that value is included within the range.

As used herein "structured to provide" or "configured to provide" means that the article has features that provide the described result.

Any direction referred to herein, such as "top," "bottom," "left," "right," "upper," "lower," "above," below," and other directions and orientations are described herein for clarity in reference to the figures and are not to be limiting of an actual device or system or use of the device or system. Many of the devices, articles or systems described herein may be used in a number of directions and orientations. Directional descriptors used herein with regard to cell culture apparatuses often refer to directions when the apparatus is oriented for purposes of culturing cells in the apparatus.

It is also noted that recitations herein refer to a component being "configured" or "adapted to" function in a particular way. In this respect, such a component is "configured" or "adapted to" embody a particular property, or function in a particular manner, where such recitations are structural recitations as opposed to recitations of intended use. More specifically, the references herein to the manner in which a component is "configured" or "adapted to" denotes an existing physical condition of the component and, as such, is to be taken as a definite recitation of the structural characteristics of the component.

As used herein, the term "cell culture" refers to keeping cells alive *in vitro.* Included within this term are continuous cell lines (e.g., with an immortal phenotype), primary cell cultures, finite cell lines (e.g., non-transformed cells), and any other cell population maintained *in vitro,* including oocytes and embryos.

As used herein, the term "*in vitro*" refers to an artificial environment and to processes or reactions that occur within an artificial environment. *In vitro* environments can include, but are not limited to, test tubes and cell cultures. The term "*in vivo*" refers to the natural environment (e.g., an animal or a cell) and to processes or reaction that occur within a natural environment.

As used herein, "long-term culture" is meant to refer to cells (e.g., but not limited to hepatocytes) that have been cultured for at least about 12 hours, optionally for at least about 24 hours, for at least about 48 hours, or for at least about 72 hours, for at least about 96 hours, at least about 7 days, at least about 14 days, at least about 21 days, or at least about 28 days. Long-term culturing facilitates the establishment of functional properties, such as metabolic pathways, within the culture.

As used herein, the term "cell culture article" means any container useful for culturing cells and includes plates, wells, flasks, multi-well plates, multi-layer flasks, transwell inserts, transwell microcavity inserts, and perfusion systems which provide an environment for cell culture.

As used herein "chamber" means a cell culture vessel which may be a flask or a dish or a well of a multi-well plate. The chambers are wells of a multi-well plate. A cell culture article may be a multi-well plate having 6, 12, 24, 96, 384 or 1536 chambers or wells. In some embodiments, each chamber includes from about 25 to about 1,000 of said microcavities.

In embodiments, a "well" is an individual cell culture environment provided in a multi-well plate format. In embodiments, a well can be a well of a 4 well plate, a 6 well plate, a 12 well plate, a 24 well plate, a 96 well plate, a 538 well plate, a 1536 well plate, or any other multi-well plate configuration.

In embodiments, a single chamber can be a well of a multi-well plate structured to constrain cells of interest to grow as a single 3D cell mass, or as a single spheroid, in that single chamber. For example, a well of a 96 well plate (wells of traditional 96 well plates) are approximately 10.67 mm deep, have a top aperture of approximately 6.86 mm and a well bottom diameter of approximately 6.35 mm.

In embodiments, "spheroid plate" means a multi-well plate having an array of single-spheroid chambers or wells.

That is, in embodiments, a multi-well plate may have multiple chambers or wells, wherein each chamber or well is configured to contain a single spheroid.

As used herein "microwell" or "microcavity" which is "structured to constrain cells of interest to grow in 3D conformation" or the like means microwells, or microcavities having dimensions or treatments, or a combination of dimensions and treatments, which encourage cells in culture to grow in 3D or spheroid conformation rather than as two dimensional sheets of cells. Treatments include treatment with low binding solutions, treatments to render the surface less hydrophobic, or treatments for sterilization, for example.

In embodiments, the "microcavity" or "microwell" can be, for example, a microwell that defines an upper aperture and a nadir, a center of the upper aperture, and a center axis between the nadir and the center of the upper aperture. In embodiments, the microcavity or microwell well is rotationally symmetrical about the axis (i.e. the sidewall is cylindrical). In some embodiments, the upper aperture defines a distance across the upper aperture of from between 250 $\mu$m to 1 mm, or any range within those measurements. In some embodiments the distance from the upper aperture to the nadir (the depth "d") is between 200 $\mu$m and 900 $\mu$m, or between 400 and 600 $\mu$m. The array of microcavities may have different geometries, for example, parabolic, hyperbolic, chevron, and cross-section geometries, or combinations thereof.

In embodiments, a well has an array of "microcavities." In embodiments, the "microcavity" can be, for example, a microwell that defines an upper aperture and a nadir, a center of the upper aperture, and a center axis between the nadir and the center of the upper aperture. In embodiments, the well is rotationally symmetrical about the axis (i.e. the sidewall is cylindrical). In some embodiments, the upper aperture defines a distance across the upper aperture of from between 250 $\mu$m to 1 mm, or any range within those measurements. In some embodiments the distance from the upper aperture to the nadir (the depth "d") is between 200 $\mu$m and 900 $\mu$m, or between 400 and 600 $\mu$m. The array of microcavities may have different geometries, for example, parabolic, hyperbolic, chevron, and cross-section geometries, or combinations thereof.

In embodiments, a "microcavity spheroid plate" means a multi-well plate having an array of wells, each well having an array of microcavities.

In embodiments, "round bottom" of a well or microcavity well can be, for example, a hemisphere, or a portion of a hemisphere, such as a horizontal section or slice of a hemisphere making up the bottom of the well or microcavity.

In embodiments, the term "3D spheroid" or "spheroid" can be, for example, a ball of cells in culture, which are not a flat two-dimensional sheet of cells. The terms "3D spheroid" and "spheroid" are used interchangeably here. In embodiments, the spheroid is comprised of a single cell type or multiple cell types, having a diameter of, for example, from about 100 to about 500 microns, more preferably from about 150 to about 400 microns, even more preferably from about 150 to about 300 microns, and most preferably from about 200 to about 250 microns, including intermediate values and ranges, depending on, for example, the types of cells in the spheroid. Spheroid diameters can be, for example, from about 200 to about 400 microns. The maximum size of a spheroid is generally constrained by diffusion considerations (for a review of spheroids and spheroid vessels see Achilli, T-M, et. al. Expert Opin. Biol. Ther. (2012) 12(10)).

As used herein a "hepatocyte" means any cell that is derived from the main parenchymal tissue of the liver. Hepatocytes can be primary hepatocyte cells that are obtained or isolated from an animal, including a human, or hepatocytes can be hepatic cell lines or primary hepatocyte derived cells.

As used herein "insert" means a cell culture well that fits into a well of a spheroid plate or a microcavity spheroid plate. The insert has sidewalls and a bottom surface defining a cavity for culturing cells. As used herein, a "transwell microcavity insert" means an insert in which the bottom surface has an array of microcavities.

As used herein "insert plate" means an insert plate containing an array of inserts structured to fit into an array of wells of a multi-well plate. As used herein, a "microcavity insert plate" means an insert plate in which each insert in the array of inserts has a bottom surface with an array of microcavities.

As used herein, "candidate compound" or the like (e.g., "compound" "compound of interest", or "drug compound") is meant to refer to any compound (exogenously administered or endogenously generated) wherein the characterization of the compound's ADME/Tox properties are desirable. Exemplary candidate compounds include xenobiotics low molecular weight therapeutic agents commonly referred to as "drugs" and other therapeutic agents, carcinogens and environmental pollutants and endobiotics such as steroids, bile acids, fatty acids and prostaglandins. A candidate compound may include drugs, including all class of action, including but not limited to: anti-neoplastics, immuno-suppressants, immune-stimulants, anti-proliferatives, anti-thrombins, anti-platelet, anti-lipid, anti-inflammatory, anti-biotics, angiogenics, anti-angiogenics, vitamins, ACE inhibitors, vasoactive substances, anti-mitotics, metello-proteinase inhibitors, NO donors, estradiols, anti-sclerosing agents, hormones, free radical scavengers, toxins, alkylating agents, alone or in combination. A candidate compound may also include, for example and not by way of limitation, biologic agents, including but not limited to: peptides, lipids, protein drugs, protein conjugates drugs, enzymes, oligonucleotides, ribozymes, genetic material, prions, virus, and bacteria.

As used herein, "clearance" is meant to refer to the volume of blood that is completely cleared of a compound (e.g., but not limited to, a drug) per unit of time. Clearance is typically measured in ml/min or ml/min/kg.

As used herein, "intrinsic clearance" or "$Cl_{int}$," is meant to refer to the ability of the liver to remove a compound (e.g., but not limited to, a drug) in the absence of flow limitations and binding to cells or proteins in the blood. Thus, intrinsic clearance is the intrinsic ability of hepatic enzymes to metabolize the drug.

As used herein, a "low clearance compound" is meant to refer to a compound that has a clearance of <5 ml/min/kg.

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that any particular order be inferred. Any recited single or multiple feature or aspect in any one claim can be combined or permuted with any other recited feature or aspect in any other claim or claims.

While various features, elements or steps of particular embodiments may be disclosed using the transitional phrase "comprising," it is to be understood that alternative embodiments, including those that may be described using the transitional phrases "consisting" or "consisting essentially of," are implied.

As previously mentioned, ADME/Tox (absorption, distribution, metabolism, excretion and toxicity) properties of a compound are critical elements for predicting the compound's clinical success and to select against drugs with problematic profiles, reduce metabolic liability, and to ultimately enable once-a-day dosing. While traditional *in vitro* systems have had some success and resulted in an increase in percentage of low clearance compounds in pharmaceutical drug discovery programs, challenges remain. The ability to use *in vitro* data to predict *in vivo* liver metabolism and toxicity is critical. Further, even for drug candidate compounds that exhibit slow metabolism, it is critical to accurately differentiate drug candidate compounds based on their predicted clearance. For example, prediction of *in vivo* hepatic clearance of a compound is important in drug discovery and development, as understanding a compound's clearance parameter is an essential factor for determining drug-half-life, oral bioavailability, dose, and dosing regimens. The ability to generate accurate *in vitro* intrinsic clearance data is as essential element in predicting *in vivo* human clearance, as an unexpected *in vivo* clearance of a drug candidate can lead to exposure issues and safety concerns.

However, current *in vitro* liver models cannot reliably and accurately predict (e.g., within 2-fold or 3-fold of actual) the *in vivo* clearance or half-life of low clearance compounds (e.g., but not limited to low clearance drug candidates). For example, due to the fact that hepatocytes containing the full complement of oxidative/reductive, hydrolytic, and conjugative drug-metabolizing enzymes present in the liver (and thus the complete set of hepatic clearance pathways), as well as the increased availability of both fresh and cryopreserved hepatocytes, *in vitro* hepatocyte suspensions are a commonly used predict *in vivo* hepatic clearance. However, hepatocyte suspensions, as well as human liver microsomes, generally under-predict *in vivo* clearance, particularly for low clearance compounds. This is largely due to the rapid loss of enzymatic activity of *in vitro* hepatocyte suspensions (typically within 4-6 hours) and human liver microsomes (typically limited to about 1-2 hours) over time, which therefore precludes the ability to accurately evaluate the metabolic stability of slowly metabolized, low clearance compounds. Rather, the use of human liver microsomes typically has a lower limit of intrinsic clearance measurements of 10 ml/min/kg, while the use of human hepatocyte suspensions typically has a lower limit of intrinsic clearance measurements of 6.3 ml/min/kg. As previously stated, low clearance candidate compounds have a clearance of <5 ml/min/kg.

The present disclosure describes, among other things, methods and labware for evaluating the interaction of a candidate compound on 3D hepatocyte spheroids for use in various biochemical and molecular biology studies, particularly ADME/Tox (absorption, distribution, metabolism, excretion and toxicity) studies. In aspects, the methods and labware can be used to evaluate the metabolism of a candidate compound in an *in vitro* 3D hepatocyte spheroid culture. The methods are performed in labware that combine 3D spheroid culture with micro-patterned design that allows for prolonged maintenance of liver cells (e.g. hepatocytes) viability and functionality, but also for multiple to several hundreds of spheroids to be treated under the same conditions and medium (e.g., during a long-term culture) for the production of sufficient materials (e.g., parent drug, drug metabolites, and DNA, RNA, and proteins from cells) and higher detection signal intensity for ADME/Tox (absorption, distribution, metabolism, excretion and toxicity) studies, all while providing a physical barrier between individual 3D spheroids to prevent any spheroid fusion during culture or testing. In some embodiments of instantly-disclosed methods, the one or more candidate compound is a low clearance compound. As previously described, a low clearance compound is a compound having a clearance of <5 ml/min/kg. Low clearance compounds include, but are not limited to, warfarin, meloxicam, tolbutamide, diazepam, alprazolam, glimepiride, prednisolone, riluzole, and voriconazole. Unlike most current *in vitro* liver models that cannot reliably predict the *in vivo* clearance or half-life of low clearance drug candidates, the instant methods allow for, among other uses, the investigation and generation of accurate *in vitro* intrinsic clearance data, and thus more accurate prediction of *in vivo* clearance, particularly with such low clearance compounds. For example, the instantly-disclosed methods that combine 3D spheroid culture with micro-patterned design generated *in vitro* intrinsic clearance data of 9 low clearance compounds that had an accuracy of prediction of *in vivo* clearance of 44% within 2-fold of actual and 67% within 3-fold of actual. In contrast, methods that utilized a 2D monolayer only generated *in vitro* intrinsic clearance data that had an accuracy of prediction *of in vivo* clearance of 33% within 2-fold of actual and 44% within 3-fold of actual for the same 9 low clearance compounds. Further, as compared to other co-culture systems, such as HepatoPac®, the instant methods do not require animal stromal cells in the 3D spheroid culture and can be completed at a significantly reduced cost. Additionally, as compared to *in vitro* relay methods, the instant methods are much less labor intensive as they do not require repeated thawing of hepatocytes and supernatant transfer.

In various embodiments, an assay method for evaluating the interaction of one or more low clearance candidate compounds with hepatocytes is disclosed. In aspects, an assay method for evaluating the metabolism of a low clearance candidate compound in an *in vitro* 3D hepatocyte spheroid culture is disclosed. The assay method comprises culturing hepatocytes in a cell culture article to form a spheroid, wherein the cell culture article comprises a chamber, the chamber comprising an array of microcavities, each microcavity structured to constrain the hepatocytes to grow in a 3D spheroid conformation. The assay method further comprises contacting the 3D spheroid hepatocytes with one or more low clearance candidate compounds. The assay method also comprises measuring the interaction of the one or more low clearance candidate compounds with the 3D hepatocyte spheroids in the *in vitro* culture, with such an interaction including but not limited to, clearance studies (uptake clearance; basolateral efflux clearance; canalicular efflux clearance; metabolic clearance), metabolite ID and metabolic stability (parent lifetime), intracellular concentration of the candidate compound, protein and gene regulation (induction/suppression) and other molecular, biochemical, and genetic analysis of the cultured 3D spheroid hepatocytes, a toxicological effect, compound kinetics, subcellular accumulation and free or total (the combination of bound and free) intracellular concentration, overall biliary clearance, P450 and transporter drug interactions, and pharmacokinetics. In embodiments, the measured *in vitro* interaction is used to predict an *in vivo* disposition of the one or more low clearance candidate compounds.

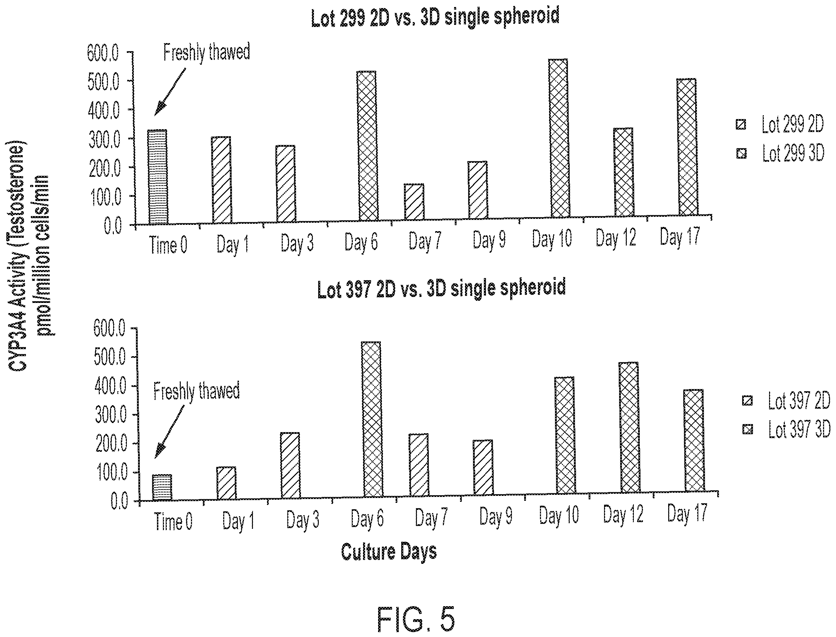

In some embodiments of the instantly-disclosed methods, the cultured cells, including but not limited to the hepatocytes that form a 3D spheroid (in aspects, with the one or more low clearance candidate compounds), are cultured as a long-term culture. In some embodiments, the cultured cells are cultured (in aspects, with the one or more low clearance candidate compounds) for at least about 12 hours, optionally for at least about 24 hours, for at least about 48 hours, or for at least about 72 hours, for at least about 96 hours, at least about 7 days, at least about 14 days, at least about 21 days, or at least about 28 days. Long-term culturing facilitates the establishment of functional properties, such as metabolic pathways, within the culture. For example, as shown in **FIG. 5**, primary human hepatocytes from two donors (Lot 299 (top) and Lot 397 (bottom) cultured in a 96-well spheroid culture plate using the instantly-disclosed labware and methods to form 3D hepatocyte spheroids maintained higher CYP3A4 activity (as activated and measured by the addition of testosterone) for an extended period of time as compared to hepatocytes cultured in 2D. In some embodiments of the instantly disclosed methods, the cultured 3D spheroid hepatocytes are functional stable for at least about 1 week, at least about 2 weeks, at least about 3 weeks, or at least about 4 weeks. Functional stability can be measured as is known in the art, including but not limited to, measurement of metabolic activity, cell function, gene expression, or a combination thereof. In some embodiments, functional stability is measured by CYP3A4 activity.

In some embodiments, each microcavity of the chamber comprises a top aperture and a liquid impermeable bottom comprising a bottom surface, wherein at least a portion of the bottom surface comprises a low-adhesion or no-adhesion material in or on the bottom surface. In some embodiments, the liquid impermeable bottom including the bottom surface is gas-permeable. In some embodiments, at least a portion of the bottom is transparent. In some embodiments, the bottom surface comprises a concave bottom surface. In some embodiments, the at least one concave bottom surface of each microcavity of the chamber includes a hemi-spherical surface, a conical surface having a taper of 30 to about 60 degrees from the side walls to the bottom surface, or a combination thereof. In some embodiments, the chamber further comprises a side wall. In some embodiments, the cell culture article includes from 1 to about 2,000 of said chambers, wherein each chamber is physically separated from any other chamber. In some embodiments, each chamber includes from about 25 to about 1,000 of said microcavities. For example, and not by way of limitation, a 6-well plate may comprise approximately 700 microcavities in one well of the 6-well plate, thus allowing for 700 spheroids (approximately 1.2 million cells in total) within one well. Similarly, a 24-well plate may comprise approximately 100-200 microcavities per well, while a 96-well plate may comprise approximately 50 microcavities per well.

Cells cultured in three dimensions, such as spheroids, can exhibit more *in vivo* like functionality than their counterparts cultured in two dimensions as monolayers. In two dimensional cell culture systems, cells can attach to a substrate on which they are cultured. However, when cells are grown in three dimensions, such as spheroids, the cells interact with each other rather than attaching to the substrate. Cells cultured in three dimensions more closely resemble *in vivo* tissue in terms of cellular communication and the development of extracellular matrices. For example, 3D spheroid culture of hepatocytes results in sustained hepatocyte drug metabolic activity and cell viability. Thus, hepatocyte 3D spheroids thus provide a superior model for ADME/Tox studies, including the investigation and generation of accurate *in vitro* intrinsic clearance data for a more accurate prediction of *in vivo* clearance, particularly with such low clearance compounds.

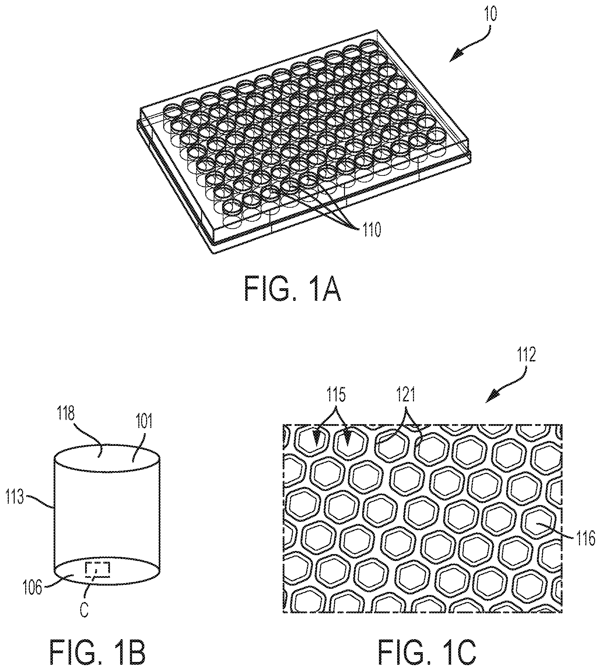

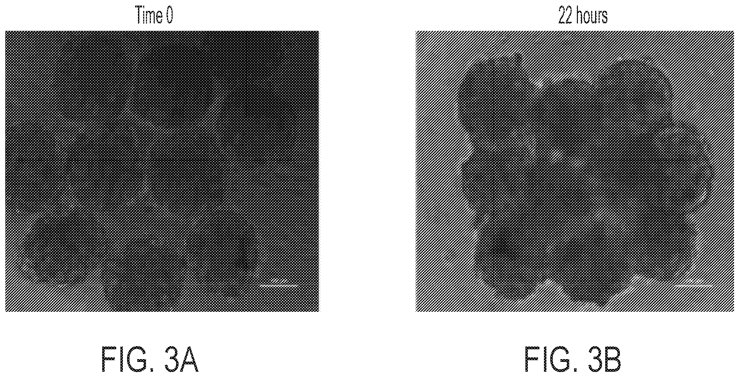

Referring now to **FIG. 1A, FIG. 1B** and **FIG. 1C,** an embodiment of a cell culture article that is a microcavity spheroid plate, in this case a 96-well microcavity spheroid plate, having an array of microcavities on the bottom surface of each well, with each microcavity structured to constrain the cultured cells to grow in a 3D spheroid conformation, to provide multiple spheroids in each of the 96 wells is shown. **FIG. 1A** illustrates a multi-well plate **10** having an array of wells **110. FIG. 1B** illustrates a single well **101** of the multi-well plate **10** of **FIG. 1A.** The single well **101** has a top aperture **118,** a liquid impermeable bottom surface **106,** and a sidewall **113**. FIG. 1C is an exploded view of the area of the bottom surface **106** of the well **101** shown in the box C in **FIG. 1B** illustrating an array of microcavities **112** in the bottom surface of the single well shown in **FIG. 1B**. Each microcavity **115** in the array of microcavities **112** has a sidewall **121** and a liquid impermeable bottom surface **116**. The microcavity spheroid plate shown in **FIG. 1A, FIG. 1B** and **FIG. 1C,** which provides an array of microcavities **112** in the bottom of each individual well **101,** can be used to grow an individual 3D spheroid in each of the microcavities of each individual well of the multi-well plate. By using this type of vessel, a user can grow a large number of spheroids in each well of a multi-well plate and thereby provide a large number of liver cell spheroids that maintain prolonged viability and functionality and that can be treated under the same culture and experimental conditions for use in an assay as provided herein. Further, this type of vessel provides a physical barrier between individual 3D spheroids to prevent any spheroid fusion during culture or testing. As shown in **FIG. 3A** and **FIG. 3B,** when spheroids made from Corning® HepatoCells (shown at time 0 in **FIG. 3A**) were manually pooled together and incubated for 22 hours, the spheroids fused together (shown in **FIG. 3B**). Fusion of the spheroids can lead to the change of exposed cell surface area ratio to total volume. Thus, the microwell design of the instant disclosure provides a physical barrier that allows for the integrity of each spheroid to be maintained during extended incubation time with testing compounds to allow for homogenous diffusion and drug metabolism activities across the well.

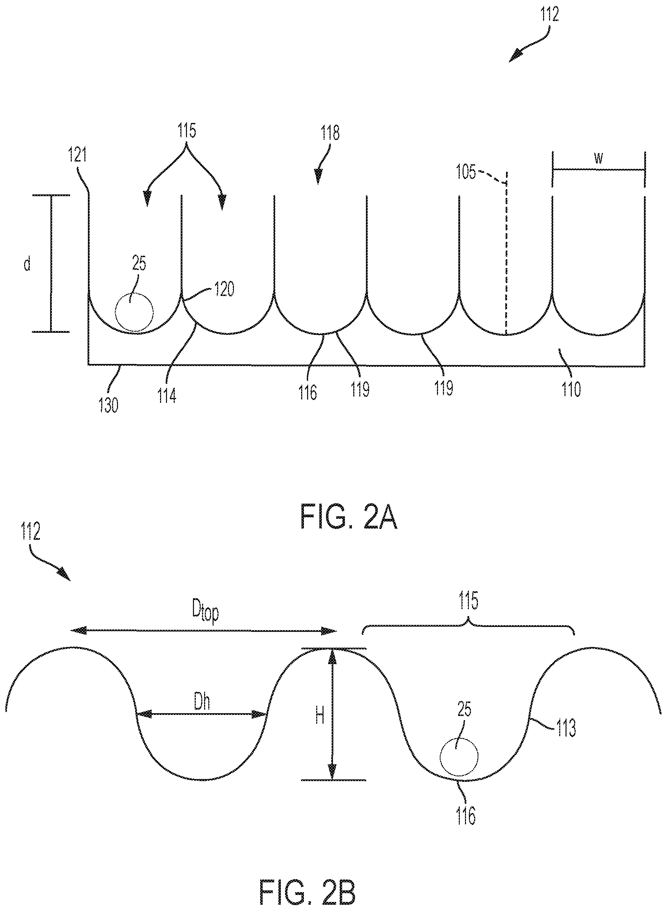

Referring now to **FIG**. 2A, an exemplary illustration of an array of microcavities **112** is shown. **FIG. 2A** illustrates microcavities **115,** each having top aperture **118,** a bottom surface **119,** a depth d, and a width w defined by sidewalls **121**. As shown in **FIG. 2A** and **FIG 2B,** the array of microcavities have a liquid impermeable, concave arcuate bottom surface **116**. In embodiments, the bottom surfaces of the microcavities can be round or conical, angled, flat bottomed, or any shape suitable for forming 3D spheroids. A rounded bottom is preferred. The round bottom **119** can have a transition zone **114** as the perpendicular sidewalls transition into a round bottom **119**. This can be a smooth or angled transition zone. In embodiments, the "microcavity" can be, for example, a microwell **115** that defines an upper aperture **118** and a nadir **116,** a center of the upper aperture, and a center axis **105** between the nadir and the center of the upper aperture. In embodiments, the well is rotationally symmetrical about the axis (i.e. the sidewall is cylindrical). In some embodiments, the upper aperture defines a distances across the upper aperture (width w) of from between 250 $\mu$m to 1 mm, or any range within those measurements. In some embodiments the distance from the upper aperture to the nadir (the depth "d") is between 200 $\mu$m and 900 $\mu$m, or between 400 and 600 $\mu$m. The array of microcavities may have different geometries, for example, parabolic, hyperbolic, chevron, and cross-section geometries, or combinations thereof. In embodiments, the microcavities may have a protective layer **130** below them to protect them from direct contact with a surface such as a lab bench or a table. In some embodiments, there may be an air space **110** provided between the bottom of the wells **119** and the protective layer. In embodiments, the air space **110** may be in communication with the external environment, or may be closed. 3D hepatocyte spheroids **25** are shown at the bottom of some individual microcavities **115**. Referring now to **FIG. 2B,** a further exemplary illustration of an array of microcavities **112** is shown. **FIG. 2B** illustrates that the array of microcavities **112** may have a sinusoidal or parabolic shape. This shape creates a rounded top edge or microcavity edge which, in embodiments, reduces the entrapment of air at a sharp corner or 90 degree angle at the top of a microcavity. As shown in **FIG. 2**B, in aspects the microcavity **115** has a top opening having a top diameter Dtop, a height from the bottom of the microcavity **116** to the top of the microcavity H, a diameter of the microcavity at a height half-way between the top of the microcavity and the bottom 116 of the microcavity $D_H$, and a sidewall **113**. In such embodiments, the bottom of the well is rounded (e.g., hemispherically round), the side walls increase in diameter from the bottom of the well to the top and the boundary between wells is rounded. As such the top of the wells does not terminate at a right angle. In some embodiments, a well has a diameter D at the half-way point (also termed $D_H$) between the bottom and top, a diameter $D_{top}$ at the top of the well and a height H from bottom to top of the well. In these embodiments, $D_{top}$ is greater than D.

In embodiments, the bottom surface of a microcavity having the at least one concave arcuate bottom surface or "cup" can be, for example, a hemi-spherical surface, a conical surface having a rounded bottom, and like surface geometries, or a combination thereof. The microcavity bottom ultimately terminates, ends, or bottoms-out in a spheroid "friendly" rounded or curved surface, such as a dimple, a pit, and like concave frusto-conicial relief surfaces, or combinations thereof. In embodiments, the at least one concave surface of each microcavity in the chamber includes a hemi-spherical surface, a conical surface having a taper of 30 to about 60 degrees from the side walls to the bottom surface, or a combination thereof. In some embodiments, the at least one concave arcuate bottom surface can be, for example, a portion of a hemisphere, such as a horizontal section or slice of a hemisphere, having a diameter of, for example, from about 250 to about 5,000 microns (i.e., 0.010 to 0.200 inch), including intermediate values and ranges, depending on, for example, the well geometry selected, the number of concave arcuate surfaces within each well, the number of wells in a plate, and like considerations. Other concave arcuate surface can have, for example, parabolic, hyperbolic, chevron, and like cross-section geometries, or combinations thereof.

In embodiments, the cell culture article comprising a chamber, each chamber comprising microcavities (e.g., a multiwell plate, a microcavity spheroid plate, a microcavity insert, a microcavity insert plate, etc.) can further comprise a low-adhesion, ultra-low adhesion, or no-adhesion coating. The coating may be, for example, on a portion of the microcavity, such as on the at least one bottom surface or on the at least one concave bottom surface of each microcavity and/or one or more sidewalls of each microcavity. Examples of non-adherent material include perfluorinated polymers, olefins, or like polymers, or mixtures thereof. Other examples include agarose, non-ionic hydrogels such as polyacrylamides, or polyethers such as polyethyleneoxide or polyols such as polyvinylalcohol, or like materials, or mixtures thereof.

In embodiments, the side wall surface (i.e., a surround) of the chamber and/or each microcavity can be, for example, a vertical cylinder or shaft, a portion of a vertical conic of decreasing diameter from the chamber top to the chamber bottom, a vertical square shaft or vertical oval shaft having a conical transition, i.e., a square or oval at the top of the well, transitioning to a conic, and ending with a bottom having at least one concave arcuate surface, i.e., rounded or curved, or a combination thereof. Other illustrative geometric examples include holey cylinders, holey conic cylinders, first cylinders then conics, and other like geometries, or combinations thereof.

One or more of, for example, a low-attachment substrate, the well curvature in the body and base portions of the microcavities, and gravity, can induce tumor cells to self-assemble into spheroids. For example, individual cells may fall to the bottom of a microcavity, adhere to each other (and not to the low-binding coated surface of the microwell) and grow into a spheroid. Hepatocytes maintain differentiated cell function indicative of a more *in* vivo-like, response relative to cells grown in a 2D monolayer. In embodiments, the spheroid can be, for example, substantially a sphere, having a diameter of, for example, from about 100 to about 500 microns, more preferably from about 150 to about 400 microns, even more preferably from about 150 to about 300 microns, and most preferably from about 200 to about 250 microns, including intermediate values and ranges, depending on, for example, the types of cells in the spheroid. Spheroid diameters can be, for example, from about 200 $\mu$m to about 400 $\mu$m (microns), and the upper diameters being constrained by diffusion considerations

In embodiments, the cell culture article, including the chamber and/or each microcavity within the chamber, can further include opaque sidewalls and/or a gas permeable and liquid impermeable bottom comprising at least one concave surface. Opaque sidewalls prevent cross-talk between wells or microwells when fluorescent imaging is employed. In some embodiments, at least a portion of the bottom comprising at least one concave surface is transparent. Cell culture articles (e.g., a microcavity spheroid plate, a microcavity insert, a microcavity insert plate, etc.) having such features can provide several advantages for the instantly-disclosed methods, including removing the need for transferring the cultured cells spheroid from one multiwall plate (in which spheroids are formed and can be visualized) to another plate for conducting assays, therefore saving time and avoiding any unnecessary disruption of the spheroid. Further, a gas-permeable bottom (e.g., well-bottoms made from a polymer having a gas permeable properties at a particular given thickness) can allow the 3D hepatocyte spheroid to receive increased oxygenation. An exemplary gas-permeable bottom can be formed from perfluorinated polymers or polymers such as poly 4-methylpentane at certain thicknesses.

Representative thickness and ranges of gas permeable polymer can be, for example, from about 0.00254 cm (0.001 inch) to about 0.0635 cm (0.025 inch), from 0.00381 cm (0.0015 inch) to about 0.0762 cm (0.03 inch), including intermediate values and ranges (where 1 inch=25,400 $\mu$m (25,400 microns); 0.000039 inches=1 $\mu$m (1 micron). Additionally or alternatively, other materials having high gas permeability, such as polydimethylsiloxane polymers, can provide sufficient gas diffusion at a thickness, for example, of up to about 2.54cm (1 inch).

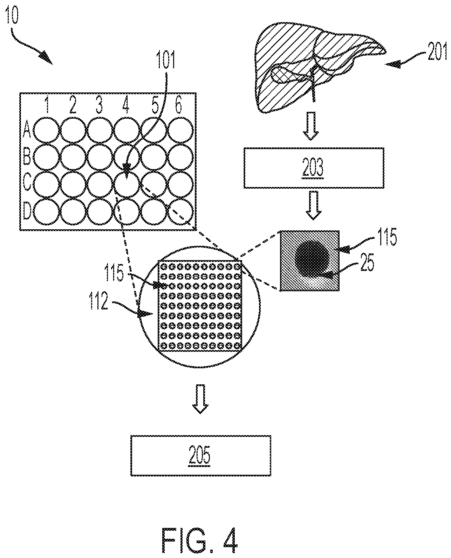

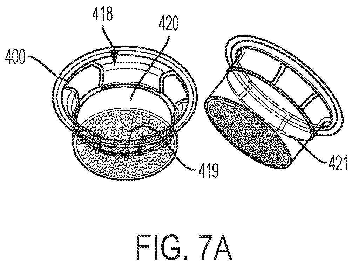

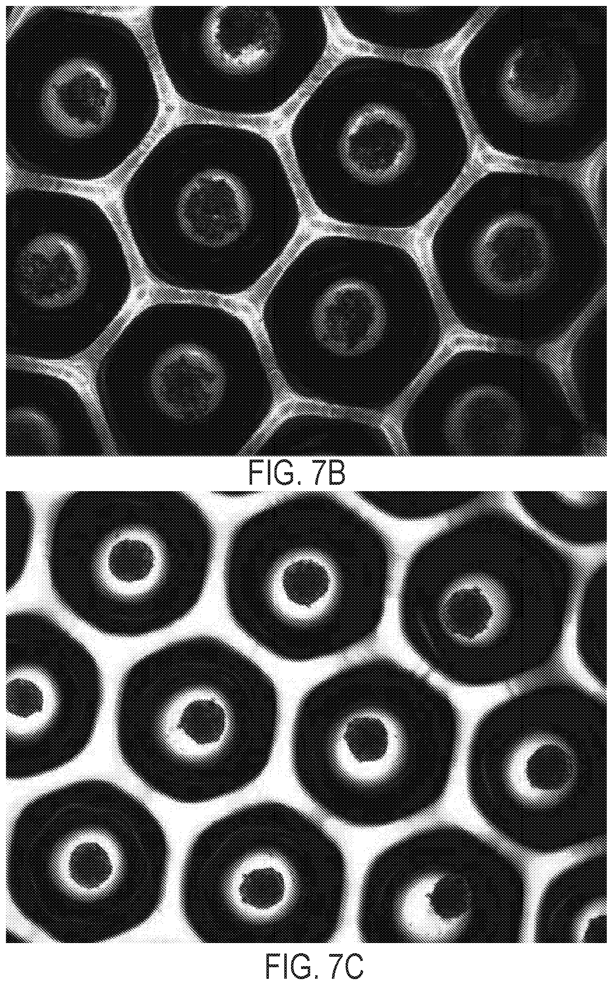

Now referring to **FIG. 4,** an illustration of an embodiment of the instantly-disclosed methods and labware for evaluating the interaction of candidate compound on 3D spheroid hepatocytes in an *in vitro* culture for use in various biochemical and molecular biology studies, particularly ADME/Tox (absorption, distribution, metabolism, excretion and toxicity) studies. In aspects, the instantly-disclosed methods and labware are used to evaluate the metabolism of one or more low clearance candidate compounds in an *in vitro* 3D hepatocyte spheroid culture. As depicted in **FIG. 4,** cells of interest **201,** such as liver cells (including but not limited to hepatocytes and/or nonparenchymal cells, which may be isolated from a liver), are grown (i.e., cultured, which may be long-term) **203** in media in microcavities of a cavity of a cell culture article (e.g., a microcavity spheroid plate, a microcavity insert, a microcavity insert plate, etc.) structured to constrain the liver cells to grow in 3D conformation. In this case and as shown, the cell culture article is a 24-well microcavity spheroid plate, and the cells of interest, such as liver cells (including but not limited to hepatocytes and/or nonparenchymal cells) are grown **203** in a well **101** of a multi-well plate **10** having an array of microcavities **112,** each microcavity **115** structured to constrain cells of interest to grow in 3D spheroid conformation **25,** which would result in the development of an array of spheroids, one in each of the microcavities in the array of microcavities on the bottom surface of a well of a multi-well plate. As the cells grow and multiply in culture, they are constrained to grow as spheroids. A spheroid **25** develops. In aspects, a microcavity insert or a microcavity insert plate can be used to culture the cells of interest to grow in 3D spheroid conformation. For example, as shown in **FIG. 7A,** an insert has a top aperture **418,** sidewalls **421** and a bottom surface **419** forming an array of microcavities **420.** The use of a microcavity insert or a microcavity insert plate can result in faster spheroid formation. For example, as shown in **FIG. 7B** (day 1 of hepatocyte culture) and **FIG. 7C** (day 3 of hepatocyte culture), a 24 well microcavity insert can be used to form hepatocyte spheroids in 3 days of culture. It should be understood that inserts are available in many configurations, including but not limited to, 6 well microcavity inserts, 12 well microcavity inserts, 24 well microcavity inserts, 48 well microcavity inserts, 96 well microcavity inserts, as well as insert plate configurations where a single plate contains multiple inserts and the multi-well insert plate is structured to insert into the complimentary array of wells in a multi-well plate.

Again referring to **FIG. 4,** once the liver cells (including but not limited to hepatocytes and/or nonparenchymal cells) develop into spheroids **25,** liver metabolism studies, including ADME/Tox (absorption, distribution, metabolism, excretion and toxicity) studies, can be performed for one or more low clearance candidate compounds **205.** For example, one or more low clearance candidate compounds can be added to the each well of the multi-well plate such that the 3D liver cell spheroids and the candidate compound, are incubated together. Then, after a suitable period of time, the interaction of the one or more low clearance candidate compounds with the cells is evaluated. Interactions of the one or more low clearance candidate compounds with the 3D liver cell spheroids (e.g., 3D hepatocyte spheroids) that can be evaluated include but are not limited to: clearance studies (uptake clearance, basolateral efflux clearance, canalicular efflux clearance, metabolic clearance); metabolite ID and metabolic stability (parent lifetime); intracellular concentration of the candidate compound; protein and gene regulation (induction/suppression) and other molecular, biochemical, and genetic analysis of the cultured 3D spheroid hepatocytes; a toxicological effect; compound kinetics; subcellular accumulation and free or total (bound+free) intracellular concentration; overall biliary clearance; P450 and transporter drug interactions; and pharmacokinetics. These interactions and effects may be measured by any appropriate means known in the art, including but not limited to, vital staining techniques, ELISA assays, immunohistochemistry, and various convention molecular, biochemical, and genetic assays. As previously described, the instant methods are performed in labware that combine 3D spheroid culture with micro-patterned design that allows for prolonged maintenance of liver cells (e.g. hepatocytes) viability and functionality, but also for multiple to several hundreds of spheroids to be treated under the same conditions (e.g., during a long-term culture) and to produce sufficient materials (e.g., parent drug, drug metabolites, and DNA, RNA, and proteins from cells) and higher detection signal intensity for the various studies listed above. The incubation period will vary depending on the one or more low clearance candidate compounds. The adjustment of the incubation time can be done in a preliminary experiment and would be well within the general skill of a person skilled in the art.

In some embodiments, an assay method for evaluating the interaction of one or more low clearance candidate compounds with hepatocytes comprises culturing hepatocytes in a cell culture article to form a spheroid, wherein the cell culture article comprises a chamber, the chamber comprising an array of microcavities, each microcavity structured to constrain the hepatocytes to grow in a 3D spheroid conformation. The assay method further includes contacting the 3D hepatocyte spheroids with one or more low clearance candidate compounds. After a suitable period of time, the assay method also includes measuring the *in vitro* intrinsic clearance of the one or more low clearance candidate compounds. This incubation period will vary depending on the candidate compound. The adjustment of the incubation time can be done in a preliminary experiment and would be well within the general skill of a person skilled in the art.

In some embodiments, the *in vitro* intrinsic clearance of the one or more candidate compounds is measured by the formation of metabolites from the one or more candidate compounds. However, to calculate intrinsic clearance from the formation (e.g., the presence and or absence) of drug metabolites, well defined clearance pathways (e.g., particular enzymes responsible for the candidate compounds clearance) and authentic metabolite standards are needed, which are not always known or available. Thus, in embodiments the *in vitro* intrinsic clearance of the one or more candidate compounds is measured by disappearance of the one or more candidate compounds. Both the measurement of the formation of metabolites from the one or more candidate compounds and/or the disappearance of the one or more candidate compounds can be measured by any means known in the art, including but not limited to mass spectrometry, liquid chromatography-mass spectrometry, liquid chromatography-tandem mass spectrometry, or high performance liquid chromatography. *In vitro* clearance can be calculated as is known in the art, such as disclosed in Chan et al. (2013) Drug Metab Dispos 41 2024-2032.

In some embodiments, the measured *in vitro* intrinsic clearance of the one or more candidate compounds is utilized to predict *in vivo* half-life of the one or more candidate compounds. In some embodiments the measured *in vitro* intrinsic clearance of the one or more candidate compounds is utilized to predict *in vivo* clearance of the one or more candidate compounds. For example, the well stirred model can be used to calculate *in vivo* hepatic clearance from the calculated *in vitro* intrinsic clearance, as shown in the Examples.

In some embodiments, the assay method comprises the step of analyzing metabolites of the one or more low clearance candidate compounds, wherein the metabolites are generated during the incubation of the 3D spheroid hepatocytes with the one or more low clearance candidate compounds. In some embodiments, analyzing metabolites of the one or more candidate compounds comprises identification of metabolites of the one or more candidate compounds generated during the incubation of the 3D spheroid hepatocytes with the one or more low clearance candidate compounds. In some embodiments, analyzing metabolites of the one or more candidate compounds comprises quantification of metabolites of the one or more candidate compounds generated during the incubation of the 3D spheroid hepatocytes with the one or more low clearance candidate compounds. The measurement of the formation of metabolites from the one or more low clearance candidate compounds, whether for identification and/or quantification of the metabolites, can be measured by any means known in the art, including but not limited to mass spectrometry, liquid chromatography-mass spectrometry, liquid chromatography-tandem mass spectrometry, or high performance liquid chromatography.

In some embodiments, the assay method comprises the step of analyzing the molecular, biochemical, or genetic effects of the one or more low clearance candidate compounds on the 3D spheroid hepatocytes. Effects on the 3D spheroid hepatocytes by the one or more low clearance candidate compounds during the incubation period can be measured by any means known in the art including genetic, metabolic or protein analysis of the cells, cell extracts, or media, such as known visualization techniques, fluorescent measurements, ELISA assays, immunohistochemistry assays, and various other known convention molecular, biochemical, and genetic assays. In some embodiments, analyzing the molecular, biochemical, or genetic effects of the one or more low clearance candidate compounds on the 3D spheroid hepatocytes comprises measuring gene and/or protein expression change during the incubation of the 3D spheroid hepatocytes with the one or more low clearance candidate compounds. In some embodiments, analyzing the molecular, biochemical, or genetic effects of the one or low clearance candidate compounds on the 3D spheroid hepatocytes comprises measuring DNA, RNA, and/or proteins produced by the 3D spheroid hepatocytes (e.g., isolated from the cells, cell extracts, and/or or media) during the incubation of the 3D spheroid hepatocytes with the one or more low clearance candidate compounds.

In some embodiments of instantly-disclosed methods, a candidate compound includes xenobiotics low molecular weight therapeutic agents commonly referred to as "drugs" and other therapeutic agents, carcinogens and environmental pollutants and endobiotics such as steroids, bile acids, fatty acids and prostaglandins. A candidate compound may include drugs, including all class of action, including by not limited to: anti-neoplastics, immuno-suppressants, immune-stimulants, anti-proliferatives, anti-thrombins, anti-platelets, anti-lipids, anti-inflammatories, anti-biotics, angiogenics, anti-angiogenics, vitamins, ACE inhibitors, vasoactive substances, anti-mitotics, metello-proteinase inhibitors, NO donors, estradiols, anti-sclerosing agents, hormones, free radical scavengers, toxins, alkylating agents, alone or in combination. A candidate compound may also include, for example and not by way of limitation, biologic agents, including but not limited to: peptides, lipids, protein drugs, protein conjugates drugs, enzymes, oligonucleotides, ribozymes, genetic material, prions, virus, and bacteria.

In some embodiments of instantly-disclosed methods, the methods comprise evaluating a plurality of candidate compounds simultaneously. In some embodiments of the instantly-disclosed methods, the methods comprise repeatedly exposing/contacting one or more candidate compounds to the 3D spheroid hepatocytes.

In some embodiments of instantly-disclosed methods, the hepatocytes can be any cell that is derived from the main parenchymal tissue of the liver. Hepatocytes can be primary hepatocyte cells that are obtained or isolated from an animal, including a human, or hepatocytes can be hepatic cell lines or primary hepatocyte derived cells. In embodiments, the cell populations can be derived from one or more species, including but not limited to, human cells, rat cells, mouse cells, monkey cells, pig cells, dog cells, guinea pig cells, fish cells. Further, the cells can be fresh or cryopreserved.

In some embodiments of the instantly-disclosed methods, non-parenchymal cells can be included with or co-cultured with the cultured hepatocytes. Non-parenchymal cells can include, but are not limited to, Kupffer cells, liver cells (including but not limited to Ito cells, sinusoidal endothelial cells, biliary duct cells) stromal cells (including but not limited to fibroblasts and pericytes), immune cells (including but not limited to T cells, neutrophils, marcrophages, dendritic cells, eosinophils, mast cells), and stem cells (including but not limited to liver progenitor cells, embryonic stem cells, and hematopoietic stem cells). These cell populations can be derived from one or more species, including but not limited to, human cells, rat cells, mouse cells, monkey cells, pig cells, dog cells, guinea pig cells, fish cells. Further, the cells can be fresh or cryopreserved. In some embodiments of the instantly-disclosed methods, there is no requirement of animal stromal cell sin the 3D spheroid culture.

In some embodiments, matrix or scaffolding can be used during culture of the hepatocytes. Thus, the hepatocytes (and possibly other co-cultured cells including non-parenchymal cells) can be embedded with, mixed with, or covered with a matrix or scaffolding during culture. Representative matrix or scaffolding includes any suitable natural or synthetic matrix or scaffolding, such as but not limited to collagen, laminin, and Matrigel®.

In some embodiments, the methods comprise culture media (e.g., comprising nutrients (e.g., proteins, peptides, amino acids), energy (e.g., carbohydrates), essential metals and minerals (e.g., calcium, magnesium, iron, phosphates, sulphates), buffering agents (e.g., phosphates, acetates), indicators for pH change (e.g., phenol red, bromo-cresol purple), selective agents (e.g., chemicals, antimicrobial agents), etc.) as are known in the art.

## EXAMPLES

The following examples are intended to illustrate specific features and aspects of the instant-disclosure and should not be construed as limiting the scope thereof.

### Example 1

### Primary Human Hepatocyte (PHH) high density (HD) spheroid culture

Pre-wetting the spheroid inserts: The pipet aid was at low disperse speed. Using a 5mL pipet and positioning the tip against the side wall at the bottom, 2mL if William's E Medium + (WEM+) medium (without FBS) was added into 6-well spheroid inserts having approximately 700 microwells/well insert. A 200ul pipet tip was used to blow away any air bubbles trapped within microwells. The plate was checked under a microscope to ensure that all air bubbles were removed.

Plating PHHs onto 6-well high density spheroid insert (~700 microwells/insert): PHH were thawed as standard procedure and resuspended in WEM plating medium. PHH cells were counted and the cell density was adjusted to 0.35e6/ml using WEM plating medium. For plating PHHs, the pipet aid was set at low disperse speed. Right before plating, a 5mL pipet was used to remove most medium from pre-wetted inserts. Even cell suspensions were prepared by gently pipetting up and down. Using a 5mL pipet, with the position the tip against the side wall at the bottom, 2mL of cell suspension (700K cells, ~1000 cells/microwell) was added to a 6-well spheroid insert. The plate was allowed to sit for 15-20min in the hood. The plates were checked under a microscopae with a 4x objective, to ensure that that a similar number of cells have settled into each microwell.

## Example 2

### 3D PHH high density spheroid drug clearance assay

Half medium change with WME+ medium: The pipet aid was at low disperse speed. Using a 5mL pipet, 1mL of old medium was slowly from each insert. A 1000$\mu$L pipet tip was positioned against the top part of the insert sidewall, and 1mL pre-warmed WME+ medium was slowly added with the medium being allowed to flow down the sidewall. After waiting 5-10 minutes between each half medium change, the half medium change was repeated 3 more times. The PHHs were checked under a microscope for spheroid formation.

Preparation of 2$\times$ clearance assay solution, with a final DMSO concentration of less than 0.2%, as shown in Table 1.

**Table 1**: 2X clearance assay solution

| Substrate | Substrate stock solution | 2X substrate concentration in WME+ medium. Final will be 0.1 $\mu$M | Assay medium volume, mL | Volume of substrate stock solution (0.2mM) to add, $\mu$L |
|---|---|---|---|---|
| Candidate compound (i.e., clearance compound) | 0.2mM in DMSO | 0.2uM | 4 ml | 4 $\mu$l |

Incubate the candidate compound(s) to be tested for clearance with the cultured spheroids: The pipet aid was set at low disperse speed. Using a 5mL pipet, 1mL of old medium was slowly removed from each insert. The 1000 $\mu$L pipet tip was positioned against the top part of the insert sidewall, and 1mL of 2X substrate solution containing the candidate compound to be tested for clearance was slowly added, with the medium allowed to flow down the sidewall. After 5-10 minutes, the culture was placed back to 37°C incubator. The timer for incubation was started at this point.

Sampling: 40$\mu$L/well stop solutions Labetalol (0.2uM IN 0.05%FA/50%ACN) was aliquoted in an assay plate and kept on ice. At the desired assay incubation time (0hr, 1 hr, 2 hrs, 4 hrs, 6 hrs, 12 hrs, 24 hrs, 36 hrs, 48 hrs, 3 days, 4 days, 5 days, 6 days, 7 days, 8 days, 9 days, 10 days, 11 days, 12 days, 13 days 14 days, 3 weeks, 4 weeks, or any value or range there between), 40 uL sample were drawn from each incubation well, mixed well with stop solution, and stored at -80°C.

LC/MS analysis: The amount of the candidate compound was measured using liquid chromatography/mass spectrometry, as is known in the art.

## Example 3

### Data Analysis and Clearance Calculations

Calculate the elimination rate constant (kei): The percentage of the candidate compound remaining in the culture was calculated by dividing the mass spectrometry peak area ratio at certain incubation times to that at time zero, as is known in the art. The natural logarithm of the percentage of the remaining candidate compound was plotted against time, and the $k_{et}$ was calculated as the absolute value from the slope.

Calculate the depletion half-lives of the candidate compounds: Once the kei was determined for the candidate compound, the depletion half-life of the candidate compound was determined by Equation 1, as is known in the art.

$$T_{1/2} = 0.692/k_{el} \qquad\qquad (1)$$

Calculate the *in vitro* clearance (Clint) of the candidate compound: The *in vitro* clearance of the candidate compounds was calculated using the *in vitro* half-lives from Equation 1 using the scaling factors of Equation 2, as is known in the art.

$$CL_{int} = \frac{Ln(2)}{t_{1/2}} \times \frac{\text{liver weight}}{\text{standard body weight}} \times \frac{\text{incubation volume (ml)}}{\text{hepatocytes /well}} \times \frac{\text{hepatocytes}}{\text{gram of liver}} \qquad (2)$$

Calculate predicted *in vivo* hepatic clearance (CL$_h$): The *in vivo* hepatic clearance was calculated from the *in vitro* clearance determined by Equation 2 using the well stirred model Equation 3, as is known in the art.

$$CL_h = \frac{Q \times f_u \times CL_{int}}{Q + (f_u \times CL_{int})} \qquad\qquad (3)$$

## Example 4

### Results

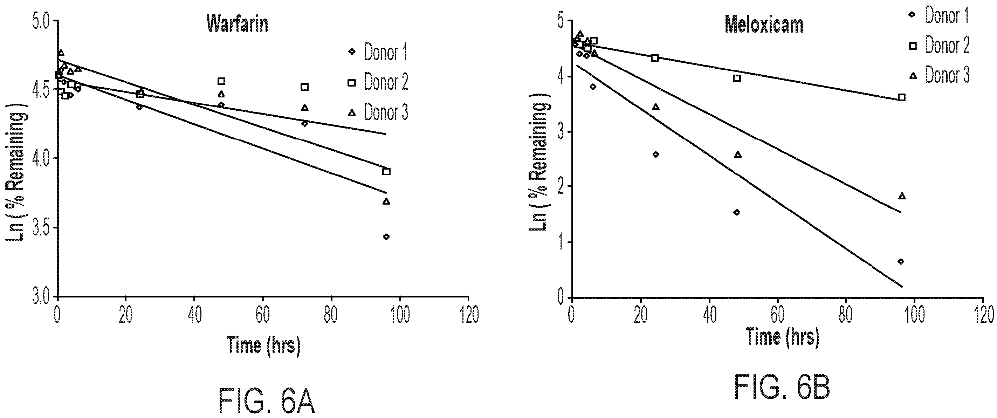

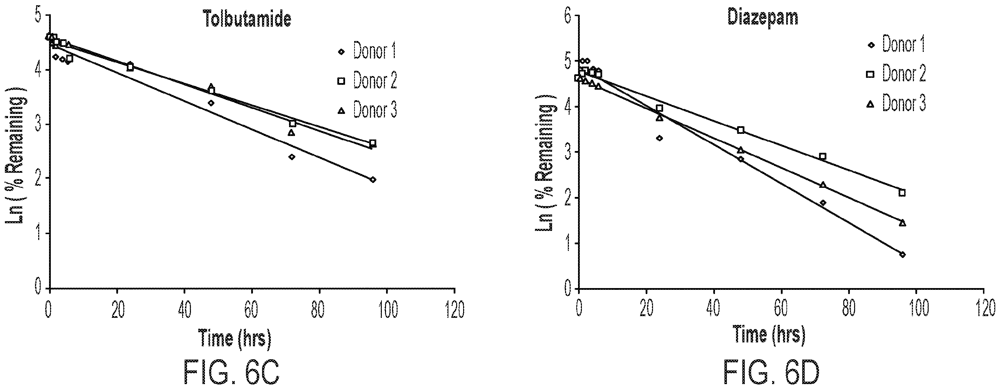

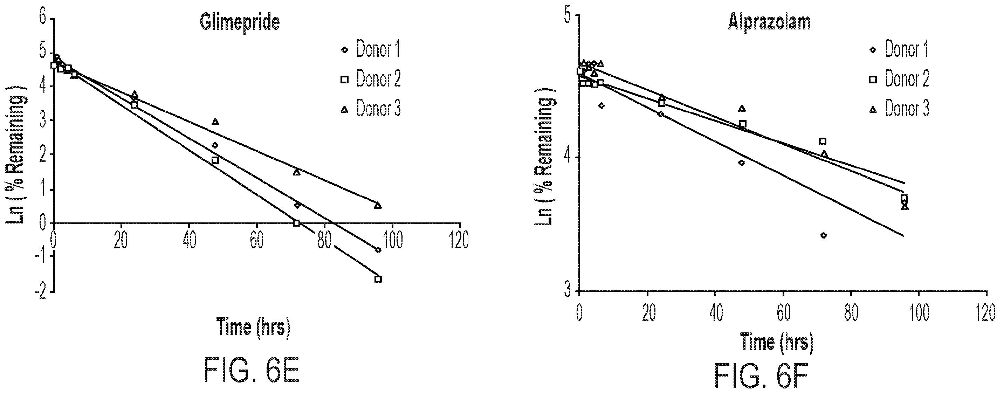

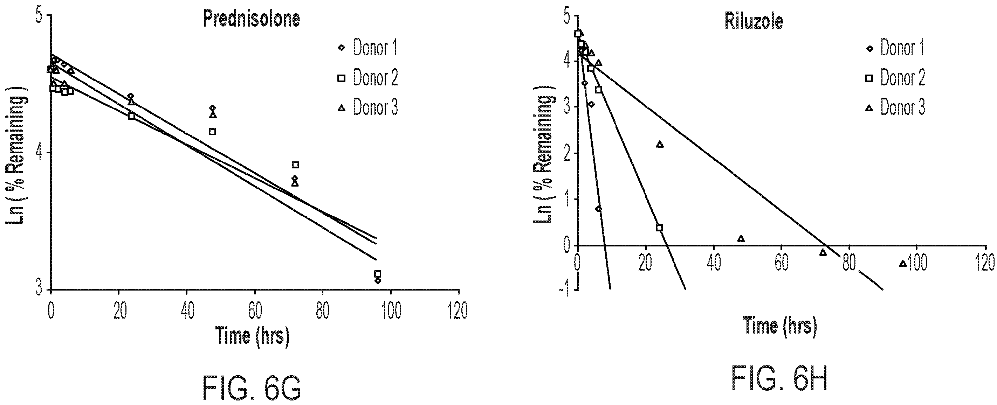

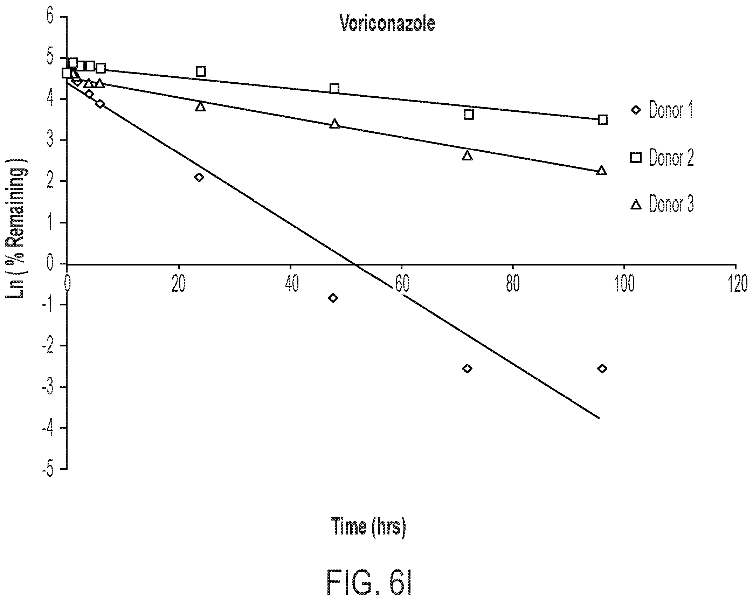

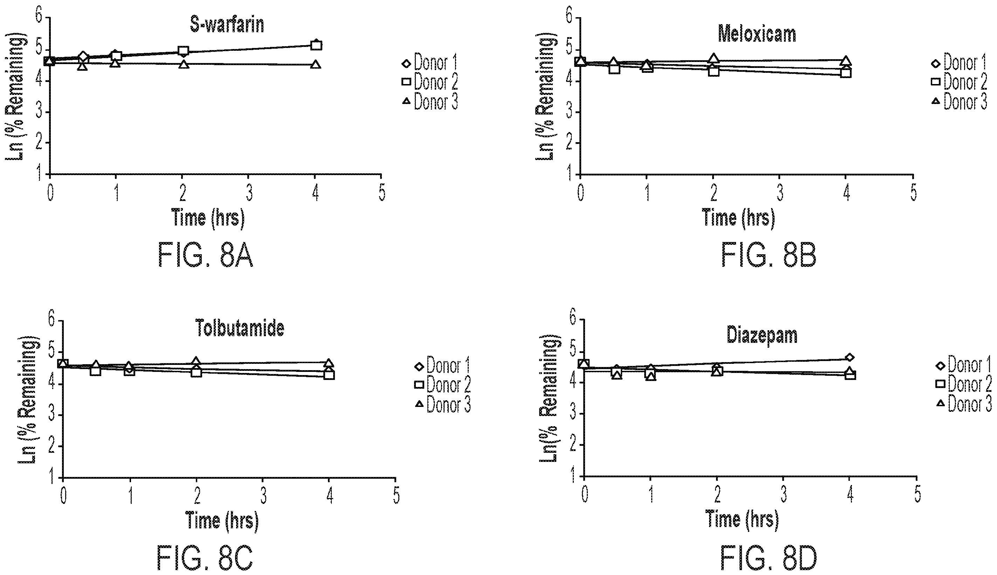

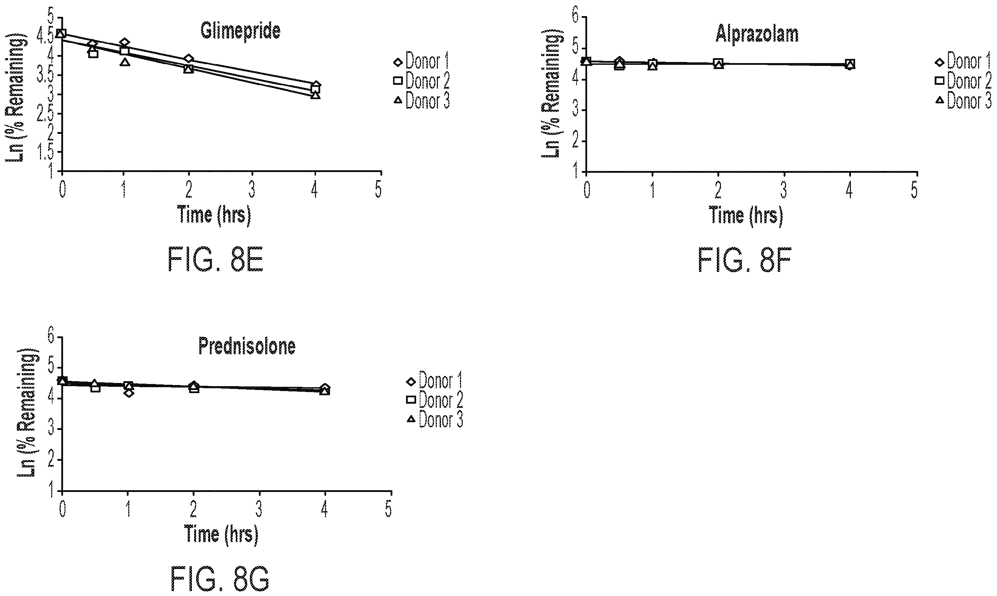

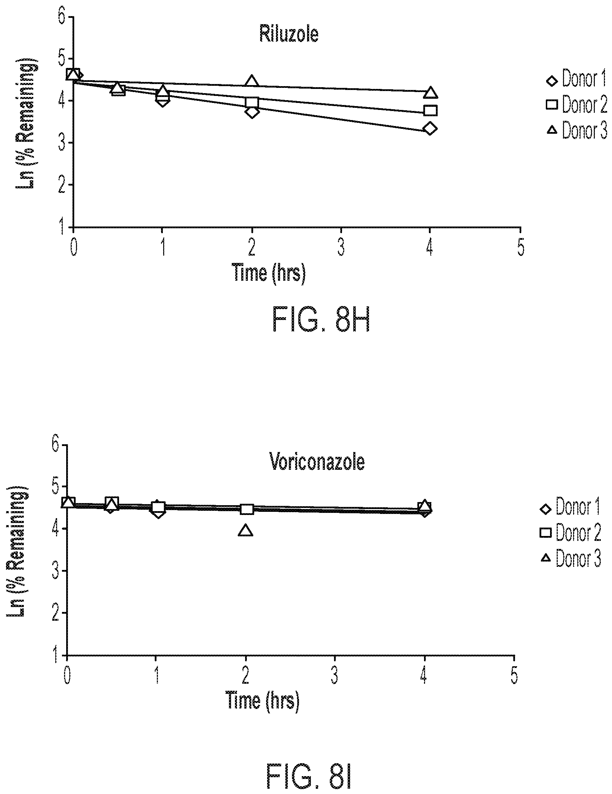

As shown in **FIG. 6A-I,** the instantly-disclosed methods and labware were able to measure classic low clearance compounds, such as warfarin **(FIG. 6A),** meloxicam **(FIG. 6B),** tolbutamide **(FIG. 6C),** diazepam (FIG. 6D), glimepiride **(FIG. 6E),** alprazolam **(FIG. 6F),** prednisolone **(FIG. 6G),** riluzole **(FIG. 6H),** and voriconazole **(FIG. 6I)**. Further, and as shown in Table 2, the instantly-disclosed methods that combine 3D spheroid culture with micro-patterned design generated *in vitro* intrinsic clearance data of 9 low clearance compounds that had an accuracy of prediction *of in vivo* hepatic clearance of 44% within 2-fold of actual and 67% within 3-fold of actual. In contrast, methods that utilized a 2D monolayer only generated *in vitro* intrinsic clearance data that had an accuracy of prediction *of in vivo* clearance of 33% within 2-fold of actual and 44% within 3-fold of actual for the same 9 low clearance compounds. Thus, unlike most current *in vitro* liver models that cannot reliably predict the *in vivo* clearance or half-life of low clearance drug candidates, the instantly-disclosed data demonstrates that the instant methods allow for, among other uses, the investigation and generation of accurate *in vitro* intrinsic clearance data of low clearance compounds, and thus more accurate prediction of *in vivo* clearance, particularly with such low clearance compounds. In contrast, PHHs grown in suspension format were unable to measure classic low clearance compounds, such as warfarin **(FIG. 8A),** meloxicam **(FIG. 8B),** tolbutamide **(FIG. 8C),** diazepam **(FIG. 8D),** glimepiride **(FIG. 8E),** alprazolam **(FIG. 8F),** prednisolone **(FIG. 8G),** riluzole **(FIG. 8H),** and voriconazole **(FIG. 8I)**.

Table 2: Predicted CL$_h$ for low clearance compounds in 2D hepatocyte culture versus 3D hepatocyte spheroid culture

| Compound | Major P450 | *In Vivo* CL nonrenal | 3D HD Spheroids |
|---|---|---|---|
| **Warfarin** | **CYP2C9** | **0.081** | 0.01 |
| **Meloxicam** | **CYP2C9** | **0.12** | 0.04 |
| **Tolbutamide** | **CYP2C9** | **0.31** | 0.22 |
| **Diazepam** | **CYP2C19** | **0.53** | 0.09 |
| **Alprazolam** | **CYP3A4** | **0.61** | 0.45 |
| **Glimepiride** | **CYP2C9** | **1.12** | 0.04 |
| **Prednisolone** | **CYP3A4** | **1.44** | 0.60 |

(continued)

| Compound | Major P450 | *In Vivo* CL nonrenal | 3D HD Spheroids |
|---|---|---|---|
| Riluzole | CYP1A2 | 2.05 | 1.62 |
| Voriconazole | CYP2C19 | 3.8 | 4.20 |
| Within 2-fold (%) | | | 44 |
| Within 3-fold (%) | | | 67 |

**Claims**

1. An assay method for evaluating the interaction of one or more candidate compounds with hepatocytes, comprising: culturing hepatocytes in a cell culture article to form a spheroid (25), wherein the cell culture article comprises a plurality of wells of a multi-well plate, each well being a chamber, each chamber comprising an array of microcavities (112), each microcavity 115) structured to constrain the hepatocytes to grow in a 3D spheroid conformation (25); wherein each microcavity 115) of the chamber comprises:
   a side wall (113, 121); a top aperture (118); and a liquid impermeable bottom (106, 116) comprising a concave surface, wherein at least a portion of the bottom (106, 116) comprises a low-adhesion or no-adhesion material in or on the concave surface;

   a) contacting the 3D spheroid hepatocytes (25) with one or more candidate compounds; and
   b) measuring the *in vitro* intrinsic clearance of the one or more candidate compounds.

2. The assay method of claim 1, wherein the cell culture article is a multi-welled plate and each well of the multi-welled plate is a chamber.

3. The assay method of claim 1 or 2, wherein the liquid impermeable bottom (106, 116) comprising the concave surface is gas-permeable.

4. The assay method of any of claims 1-3, wherein the side walls (113, 121) are opaque.

5. The assay method of any one of claims 1-4, wherein at least a portion of the bottom (106, 116) is transparent.

6. The assay method of any of claims 1-3, wherein the concave surface comprises a hemi-spherical surface (25), a conical surface having a taper of 30 to about 60 degrees from the side walls (113, 121) to the bottom surface (106, 116), or a combination thereof.

7. The assay method of claims 1-6, wherein the side wall (113, 121) surface comprises a vertical cylinder, a portion of a vertical conic of decreasing diameter form the chamber's top to bottom surface (106, 116), a vertical square shaft having a conical transition to the concave bottom surface (106, 116), or a combination thereof.

8. The assay method of any one of claims 1-7, wherein the cell culture article comprises from 1 to about 2,000 of said chambers, wherein each chamber is physically separated from any other chamber.

9. The assay method of any one of claims 1-8, wherein the *in vitro* intrinsic clearance of the one or more low clearance candidate compounds is measured by disappearance of the one or more candidate compounds; or wherein the *in vitro* intrinsic clearance of the one or more candidate compounds is measured by the formation of metabolites from the one or more candidate compounds.

10. The assay method of any one of claims 1-8, wherein the measured *in vitro* intrinsic clearance of the one or more candidate compounds is utilized to predict *in vivo* half-life of the one or more candidate compounds; or wherein the measured *in vitro* intrinsic clearance of the one or more candidate compounds is utilized to predict *in vivo* clearance of the one or more candidate compounds.

11. The assay method of any one of claims 1-10, wherein the one or more candidate compounds is a low clearance candidate compound.

12. The assay method of any one of claims 1-11, wherein the hepatocytes comprise primary human hepatocytes or a hepatocyte cell line.

13. The assay method according to any one of claims 1-12, further comprising evaluating a plurality of candidate compounds simultaneously.

14. The assay method of any one of claims 1-13, wherein the 3D spheroid hepatocytes (25) are functionally stable for at least 3 weeks.

15. The assay method of any one of claims 1-14, wherein the functional stability of the 3D spheroid hepatocytes (25) is determined by measuring metabolic activity, cell function, gene expression, or a combination thereof.

16. The assay method of any one of claims 1-15, wherein the functional stability is measured by CYP3A4 activity.


**Patentansprüche**

1. Testverfahren zum Bewerten der Wechselwirkung einer oder mehrerer Kandidatenverbindungen mit Hepatozyten, umfassend:

   Kultivieren von Hepatozyten in einem Zellkulturartikel, um ein Sphäroid (25) zu bilden, wobei der Zellkulturartikel eine Vielzahl von Vertiefungen einer Mikrotiterplatte umfasst, wobei jede Vertiefung eine Kammer ist, jede Kammer eine Anordnung von Mikrokavitäten (112) umfasst, jede Mikrokavität (115) so strukturiert ist, dass sie die Hepatozyten zwingt, in einer 3D-Sphäroid-Form (25) zu wachsen; wobei jede Mikrokavität (115) der Kammer Folgendes umfasst: eine Seitenwand (113, 121); eine obere Öffnung (118); und einen flüssigkeitsundurchlässigen Boden (106, 116), der eine konkave Oberfläche umfasst, wobei mindestens ein Teil des Bodens (106, 116) in oder auf der konkaven Oberfläche ein Material mit geringer oder ohne Haftung umfasst;

   a) Inkontaktbringen der 3D-Sphäroid-Hepatozyten (25) mit einer oder mehreren Kandidatenverbindungen; und
   b) Messen der intrinsischen *In-vitro*-Clearance der einen oder mehreren Kandidatenverbindungen.

2. Testverfahren nach Anspruch 1, wobei der Zellkulturartikel eine Platte mit mehreren Vertiefungen ist und jede Vertiefung der Platte mit mehreren Vertiefungen eine Kammer ist.

3. Testverfahren nach Anspruch 1 oder 2, wobei der flüssigkeitsundurchlässige Boden (106, 116), der die konkave Oberfläche umfasst, gasdurchlässig ist.

4. Testverfahren nach einem der Ansprüche 1-3, wobei die Seitenwände (113, 121) undurchsichtig sind.

5. Testverfahren nach einem der Ansprüche 1-4, wobei mindestens ein Teil des Bodens (106, 116) transparent ist.

6. Testverfahren nach einem der Ansprüche 1-3, wobei die konkave Oberfläche eine hemisphärische Oberfläche (25), eine konische Oberfläche mit einer Verjüngung von 30 bis etwa 60 Grad von den Seitenwänden (113, 121) zu der Bodenoberfläche (106, 116) oder eine Kombination davon umfasst.

7. Testverfahren nach einem der Ansprüche 1-6, wobei die Oberfläche der Seitenwand (113, 121) einen vertikalen Zylinder, einen Teil eines vertikalen Kegels mit abnehmendem Durchmesser von der oberen zu der Bodenoberfläche (106, 116) der Kammer, einen vertikalen quadratischen Schaft mit einem konischen Übergang zu der konkaven Bodenoberfläche (106, 116) oder eine Kombination davon umfasst.

8. Testverfahren nach einem der Ansprüche 1-7, wobei der Zellkulturartikel von 1 bis etwa 2.000 der Kammern umfasst, wobei jede Kammer physisch von jeder anderen Kammer getrennt ist.

9. Testverfahren nach einem der Ansprüche 1-8, wobei die intrinsische *In-vitro*-Clearance der einen oder mehreren Kandidatenverbindungen mit geringer Clearance durch das Verschwinden der einen oder mehreren Kandidatenverbindungen gemessen wird; oder
   wobei die intrinsische *In-vitro*-Clearance der einen oder mehreren Kandidatenverbindungen durch die Bildung von Metaboliten von der einen oder den mehreren Kandidatenverbindungen gemessen wird.

10. Testverfahren nach einem der Ansprüche 1-8, wobei die gemessene intrinsische *In-vitro*-Clearance der einen oder mehreren Kandidatenverbindungen verwendet wird, um die *In-vitro*-Halbwertszeit der einen oder mehreren Kandidatenverbindungen vorherzusagen; oder
wobei die gemessene intrinsische *In-vitro*-Clearance der einen oder mehreren Kandidatenverbindungen verwendet wird, um die *In-vivo*-Clearance der einen oder mehreren Kandidatenverbindungen vorherzusagen.

11. Testverfahren nach einem der Ansprüche 1-10, wobei die eine oder mehreren Kandidatenverbindungen eine Kandidatenverbindung mit geringer Clearance ist.

12. Testverfahren nach einem der Ansprüche 1-11, wobei die Hepatozyten primäre menschliche Hepatozyten oder eine Hepatozytenzelllinie umfassen.

13. Testverfahren nach einem der Ansprüche 1-12, ferner gleichzeitiges Bewerten einer Vielzahl von Kandidatenverbindungen umfassend.

14. Testverfahren nach einem der Ansprüche 1-13, wobei die 3D-Sphäroid-Hepatozyten (25) mindestens 3 Wochen lang funktionell stabil sind.

15. Testverfahren nach einem der Ansprüche 1-14, wobei die funktionelle Stabilität der 3D-Sphäroid-Hepatozyten (25) durch Messen der Stoffwechselaktivität, der Zellfunktion, der Genexpression oder einer Kombination davon bestimmt wird.

16. Testverfahren nach einem der Ansprüche 1-15, wobei die funktionelle Stabilität anhand der CYP3A4-Aktivität gemessen wird.

## Revendications

1. Procédé d'analyse permettant d'évaluer l'interaction d'un ou de plusieurs composés candidats avec des hépatocytes, comprenant :
la culture d'hépatocytes dans un article de culture cellulaire pour former un sphéroïde (25), dans lequel l'article de culture cellulaire comprend une pluralité de puits d'une plaque à puits multiples, chaque puits étant une chambre, chaque chambre comprenant un réseau de microcavités (112), chaque microcavité (115) étant structurée pour contraindre les hépatocytes à se développer dans une conformation sphéroïde 3D (25) ; dans lequel chaque microcavité (115) de la chambre comprend :
une paroi latérale (113, 121) ; une ouverture supérieure (118) ; et un fond imperméable aux liquides (106, 116) comprenant une surface concave, dans lequel au moins une partie du fond (106, 116) comprend un matériau à faible adhérence ou sans adhérence dans ou sur la surface concave ;

   a) la mise en contact des hépatocytes sphéroïdes 3D (25) avec un ou plusieurs composés candidats ; et
   b) la mesure de la clairance intrinsèque *in vitro* du ou des composés candidats.

2. Procédé d'analyse de la revendication 1, dans lequel l'article de culture cellulaire est une plaque à puits multiples et chaque puits de la plaque à puits multiples est une chambre.

3. Procédé d'analyse de l'une des revendications 1 ou 2, dans lequel le fond imperméable aux liquides (106, 116) comprenant la surface concave est perméable au gaz.

4. Procédé d'analyse de l'une quelconque des revendications 1 à 3, dans lequel les parois latérales (113, 121) sont opaques.

5. Procédé d'analyse de l'une quelconque des revendications 1 à 4, dans lequel au moins une partie du fond (106, 116) est transparente.

6. Procédé d'analyse de l'une quelconque des revendications 1 à 3, dans lequel la surface concave comprend une surface hémisphérique (25), une surface conique comportant une conicité de 30 à environ 60 degrés des parois latérales (113, 121) jusqu'à la surface de fond (106, 116), ou une combinaison de celles-ci.

**7.** Procédé d'analyse de l'une des revendications 1 à 6, dans lequel la surface de la paroi latérale (113, 121) comprend un cylindre vertical, une partie d'un cône vertical de diamètre décroissant de la surface supérieure vers la surface de fond (106, 116) de la chambre, une tige carrée verticale comportant une transition conique vers la surface de fond concave (106, 116), ou une combinaison de ceux-ci.

**8.** Procédé d'analyse de l'une quelconque des revendications 1 à 7, dans lequel l'article de culture cellulaire comprend de 1 à environ 2000 desdites chambres, dans lequel chaque chambre est physiquement séparée de toute autre chambre.

**9.** Procédé d'analyse de l'une quelconque des revendications 1 à 8, dans lequel la clairance intrinsèque *in vitro* du ou des composés candidats à faible clairance est mesurée par la disparition du ou des composés candidats ; ou dans lequel la clairance intrinsèque *in vitro* du ou des composés candidats est mesurée par la formation de métabolites à partir du ou des composés candidats.

**10.** Procédé d'analyse de l'une quelconque des revendications 1 à 8, dans lequel la clairance intrinsèque *in vitro* mesurée du ou des composés candidats est utilisée pour prédire la demi-vie *in vivo* du ou des composés candidats ; ou dans lequel la clairance intrinsèque *in vitro* mesurée du ou des composés candidats est utilisée pour prédire la clairance *in vivo* du ou des composés candidats.

**11.** Procédé d'analyse de l'une quelconque des revendications 1 à 10, dans lequel le ou les composés candidats sont des composés candidats à faible clairance.

**12.** Procédé d'analyse de l'une quelconque des revendications 1 à 11, dans lequel les hépatocytes comprennent des hépatocytes humains primaires ou une lignée cellulaire d'hépatocytes.

**13.** Procédé d'analyse de l'une quelconque des revendications 1 à 12, comprenant en outre l'évaluation simultanée d'une pluralité de composés candidats.

**14.** Procédé d'analyse de l'une quelconque des revendications 1 à 13, dans lequel les hépatocytes sphéroïdes 3D (25) sont fonctionnellement stables pendant au moins 3 semaines.

**15.** Procédé d'analyse de l'une quelconque des revendications 1 à 14, dans lequel la stabilité fonctionnelle des hépatocytes sphéroïdes 3D (25) est déterminée en mesurant l'activité métabolique, la fonction cellulaire, l'expression génétique ou une combinaison de celles-ci.

**16.** Procédé d'analyse de l'une quelconque des revendications 1 à 15, dans lequel la stabilité fonctionnelle est mesurée par l'activité CYP3A4.

FIG. 1A

FIG. 1B

FIG. 1C

FIG. 2A

FIG. 2B

21

FIG. 3A

FIG. 3B

FIG. 4

FIG. 5

Meloxicam

FIG. 6B

Warfarin

FIG. 6A

FIG. 6C

FIG. 6D

EP 3 765 848 B1

FIG. 6E

FIG. 6F

EP 3 765 848 B1

Riluzole

FIG. 6H

Prednisolone

FIG. 6G

FIG. 6I

FIG. 7A

FIG. 7B

FIG. 7C

FIG. 8A

FIG. 8B

FIG. 8C

FIG. 8D

FIG. 8F

FIG. 8E

FIG. 8G

FIG. 8H

FIG. 8I

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62642447

**Non-patent literature cited in the description**

- **MATTHEW G. BARON**. *PLOS ONE*, 03 January 2017, vol. 12 (1)
- **ACHILLI, T-M**. *Expert Opin. Biol. Ther.*, 2012, vol. 12 (10)
- **CHAN et al.** *Drug Metab Dispos*, 2013, vol. 41, 2024-2032